# EUROPEAN PATENT SPECIFICATION

(11) **EP 4 105 314 B1**
(45) Date of publication and mention of the grant of the patent: **10.01.2024**
(21) Application number: 22175445.0
(22) Date of filing: 25.05.2022
(51) Int. Cl.: C12M 1/107, C12M 1/00

(54) **DIGESTER WITH BIOMASS EXTRACTION SYSTEM**
FAULBEHÄLTER MIT EINEM SYSTEM ZUR EXTRAKTION VON BIOMASSE
DIGESTEUR AVEC SYSTÈME D'EXTRACTION DE BIOMASSE

(30) Priority: 09.06.2021 IT 202100014993
(43) Date of publication of application: 21.12.2022
(73) Proprietor: Alvus S.r.l., 39100 Bolzano BZ (IT)
(72) Inventor: Erckert, Christof, 39100 Bolzano BZ (IT); Poda, Marco, 39100 Bolzano BZ (IT)
(74) Representative: Perani & Partners S.p.A.

(56) References cited:
- WO-A2-98/04729
- CN-A- 107 099 449
- CN-Y- 200 996 369
- DE-A1-102008 038 262
- GB-A- 1 028 822

## Description

### Technical Field

The present invention relates to a digester of a biogas production plant having a biomass extraction system.

### State of the Art

Generally, digesters comprise a reservoir defined by a tank and an associated membrane within which biogas is generated from biomass contained within the tank. The latter is defined by a side wall projecting from a base associated with the ground. Such side wall has thicknesses in the order of 5-20 metres and heights in the order of 8-12 metres when built in reinforced earth, and thicknesses in the order of 50 cm and heights in the order of 7-8 metres when built in concrete. In addition, the side walls of digesters are made of structurally resistant materials such as reinforced concrete, concrete, steel or plain earth. Consequently, the base also has structural features suitable for supporting a wall having the aforesaid characteristics. Such known digesters include circuits for the extraction, reinsertion and integration of biomass in order to allow for its management and mixing within the tank. Given the characteristics of the base and side wall, such extraction circuits provide piping laid during the construction of the digester itself. In detail, the pipes are arranged underneath the base and side wall or along the base and through the thickness of the side wall. Such pipes allow fluid communication between the inside of the tank and the outside. In addition, active pumping systems are also provided on these pipes to take the biomass from the bottom of the tank and re-introduce it/integrate it into the tank. Document WO98/04729 A1 discloses a base associated with a supporting ground a side wall extended between a lower portion associated with the base and an upper portion spaced from the lower portion along a height direction perpendicular to the base, said side wall with the base defining a tank configured to receive inside it a quantity of biomass for generating biogas; a biomass extraction system configured to transport the biomass from the tank, the extraction system comprising- an extraction pipe configured to transport the biomass from inside the tank to outside the tank, said extraction pipe extending between a first end position arranged near the lower portion and a second end position in proximity to the upper portion;- pumping means associated with the extraction pipe and configured to extract the biomass from the tank via the extraction pipe; whereby the extraction pipe extends between the first end portion and the second end portion along an inner surface of the side wall facing the inside of the tank.

### Problems of the Prior Art

It should be noted that the known biogas production digesters have several problems associated with the extraction circuits. In fact, the pipes of such circuits have problems of management and integration once the base and side wall have been built as they are located below a structure and are difficult to reach. As a result, maintenance and inspection can take a lot of time to operators thus reducing biogas production. In addition, any ruptures in these pipes, apart from being difficult to detect and repair, may lead to the digester being blocked until it is repaired. In other cases, however, such as digesters whose side wall is made of concrete or steel, the pipes are passed through the side wall by drilling it. This involves additional operations during construction that may lead to longer pipe-laying times as well as the same problems listed above in the event of maintenance and breakages.

Finally, having to pass through base and sidewalls, the extraction circuits involve the use of long pipes and thus a subsequent increase in costs related to the materials and energy needed by the pumping systems to move the biomass along these pipes.

### Object of the Invention

The object of the invention is to provide a biogas production digester capable of overcoming the above mentioned drawbacks of the prior art.

In particular, it is an object of the present invention to provide a reliable and cost-effective biomass extraction system for digesters capable of optimising the extraction of biomass and ease its integration on the digester.

The defined technical task and the specified objects are substantially achieved by a digester as defined by claim 1.

### Advantages of the invention

Advantageously, the biomass extraction system of the present invention allows to facilitate maintenance operations.

Advantageously, the biomass extraction system of the present invention allows to reduce the costs associated with the use of pipes and ducts.

Advantageously, the biomass extraction system of the present invention allows to facilitate biomass extraction operations.

Advantageously, the biomass extraction system of the present invention allows to facilitate site operations during the digester construction.

Advantageously, the biomass extraction system of the present invention avoids having to drill holes in the side walls for piping, thus reducing the time required for constructing the digester and improving the relative integration of the system into already constructed digesters. The extraction system of the present invention may thereby be applied to any type of digester, without having to drill holes in the walls and to act in a complicated way on construction works. The present system is therefore applicable not only to reinforced earth digesters, but also to those made of concrete, steel, and for simple earth lagoons.

Advantageously, the biomass extraction system of the present invention allows to save energy, if compared to conventional systems, by reducing the length of extraction pipes.

### BRIEF DESCRIPTION OF THE DRAWINGS

Further characteristics and advantages of the present invention will become more apparent from the approximate and thus non-limiting description of a preferred, but not exclusive, embodiment of a biogas production digester, as illustrated in the accompanying drawings:

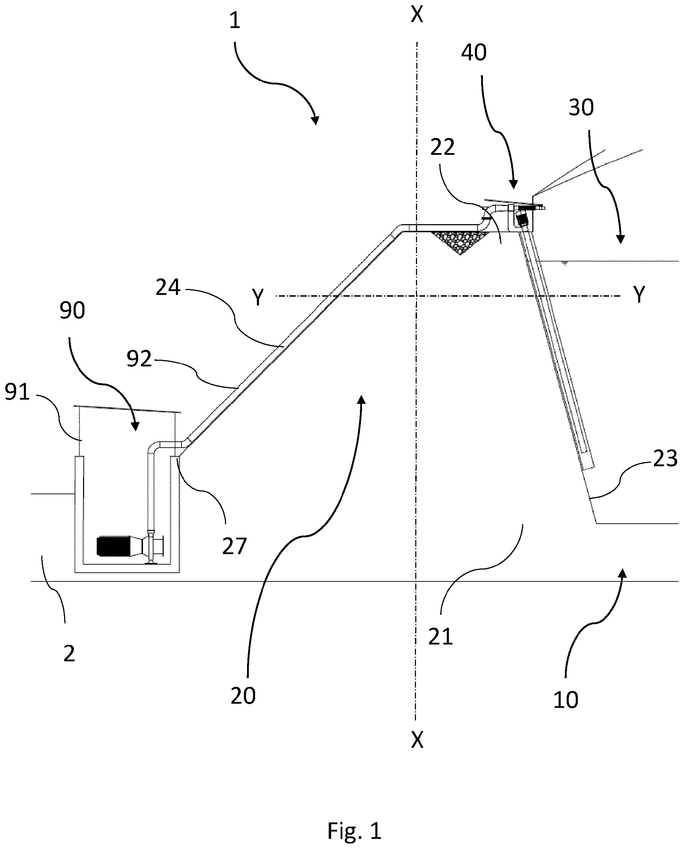

- Figure 1 shows a first section view of the digester for biogas production according to an embodiment of the present invention;
- Figure 2 shows a second section view of the digester of Figure 1;
- Figure 3 shows a third section view of the digester of Figure 1;

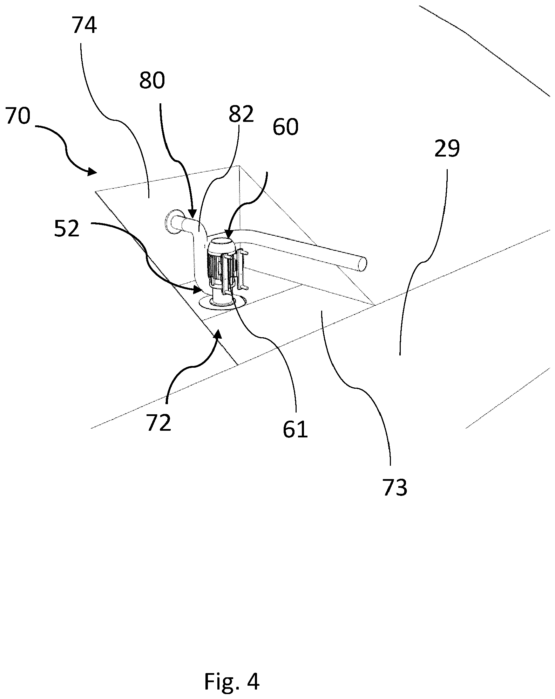

- Figure 4 shows a perspective view of the digester of Figure 1 with some parts removed to better show others;

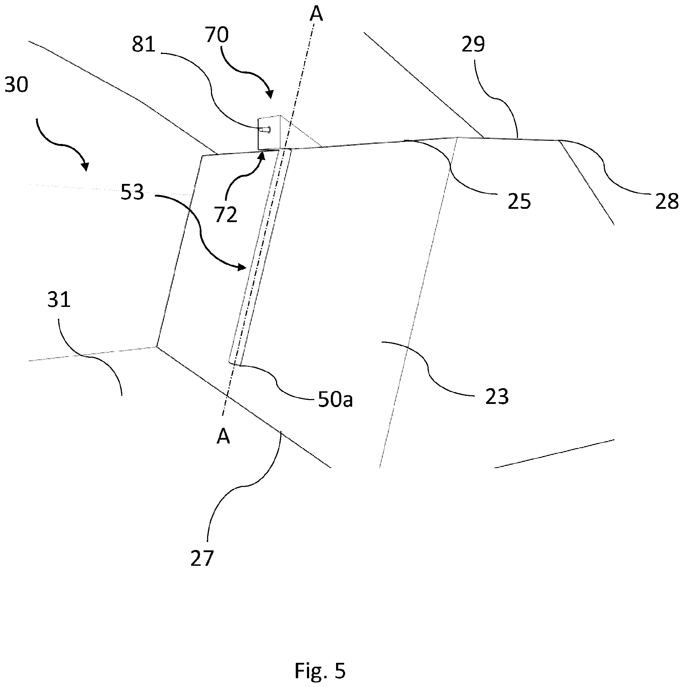

- Figure 5 shows a perspective view of the digester of Figure 1 with some parts removed to better show others;

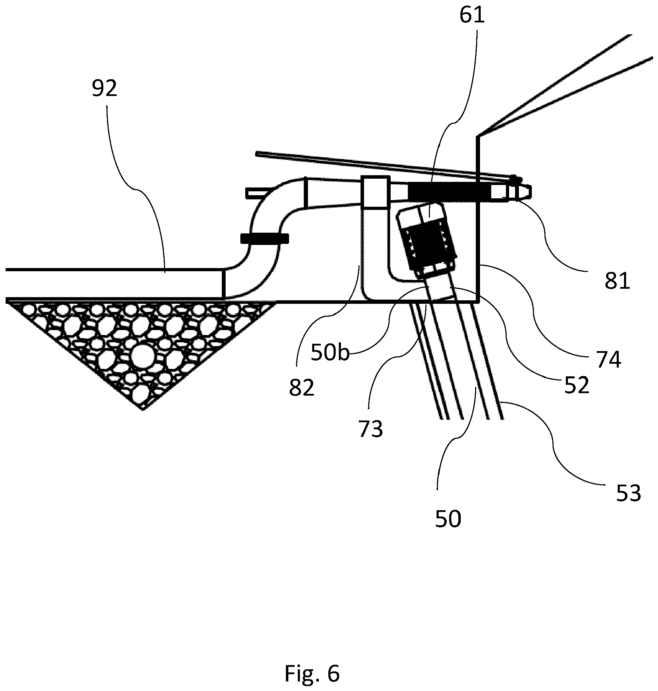

- Figure 6 shows a section view of a detail of the digester of Figure 1.

### DETAILED DESCRIPTION

The present invention relates to a digester globally referred to as 1 in the figures. Such a digester 1 is used in biogas production plants. It should be noted that for the purposes of the present invention, biogas is obtained by bacterial fermentation in anaerobiosis (absence of oxygen) of biomass from: decaying plant waste, livestock slurry or sewage sludge or agro-industrial waste or other organic waste. It is worth noting that the plant also allows to obtain bio-fertilisers (solid and liquid) in addition to biogas. Fermentation and subsequent production of biogas and/or bio-fertilisers takes place in one or more digesters and storage reservoirs. In fact, the plants also comprise one or more storage tanks configured to receive the processed biomass coming out of digesters 1 or raw biomass before being fed into digesters 1.

The digester 1 comprises a base 10 associated with a supporting ground 2. Specifically, the base 10 is substantially parallel to the supporting ground 2 and is configured to at least partially support the digester 1. Preferably, the base 10 is made of earth following appropriate levelling and/or concrete. More preferably, the base 10 represents the foundations of the digester.

The digester 1 comprises a side wall 20 extended between a lower portion 21 associated with the base 10 and an opposite upper portion 22 spaced from the lower portion 21 along a height direction X-X perpendicular to the base 10. It is worth noting that, according to a preferred embodiment, the side wall 20 is made of reinforced earth, reinforced concrete or a combination of both.

The side wall 20 with the base 10 defines a tank 30 configured to receive therein a quantity of biomass 3 used to generate biogas. It should be noted that this tank 30 may be used for making both the reservoirs for the digester 1, as illustrated below, and for storage reservoirs. The tank 30 may thereby be adapted to the needs of a plant. Each tank 30 comprises a tank base 31 and a tank side wall 32 rising from said tank base 31, preferably transverse to the tank base 31. The side wall 32 with the tank base 31 defines a cavity 33 configured to contain therein at least partially the biomass. It is worth noting that the cavity 33, for instance, may be partially occupied by gaseous substances depending on the filling of the tank with biomass.

The side wall 20 has an inner surface 23 facing the inside of the tank 30 and an opposite outer surface 24. The inner surface 23 of the side wall 20 of the digester 1 defines the tank side wall 32 while the base 20 of the digester defines the tank base 31.

The side wall 20 extends between the inner surface 23 and the outer surface 24 along a thickness direction Y-Y perpendicular to the height direction X-X. More preferably, the side wall 20 at the relative upper portion 22 comprises a supporting surface 29 connected to the inner surface 23 and to the outer surface 24 and extending along a thickness direction Y-Y. Said supporting surface 29 is substantially flat and parallel to the base 20.

It is worth noting that the inner surface 23 and outer surface 24 define an inner upper edge 25 and an outer upper edge 26 at the upper portion 22 and an inner lower edge 27 and an outer lower edge 28 at the lower portion 21, respectively. Specifically, the inner upper edge 25 delimits a respective opening of the tank cavity 33 spaced from the respective tank base 31 along the height direction X-X. By contrast, the inner lower edge 27 delimits the profile of the tank base 31. Preferably, the perimeter supporting surface 29 extends between the perimeter inner upper edge 25 and the perimeter outer upper edge 27.

The digester 1 comprises a biomass extraction system 40 configured to extract biomass 3 from the tank 30 and eventually reintroduce it by means of an irrigation apparatus 80 as explained hereinafter.

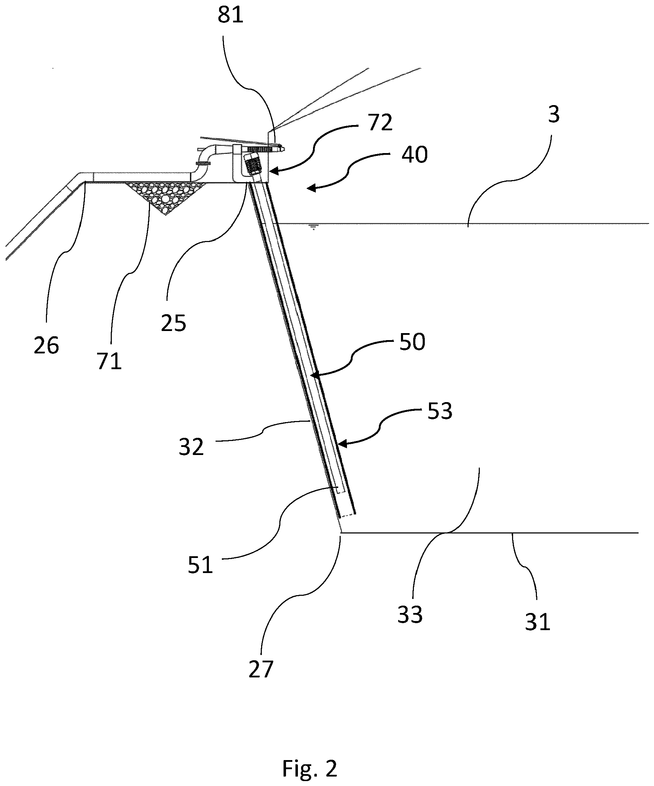

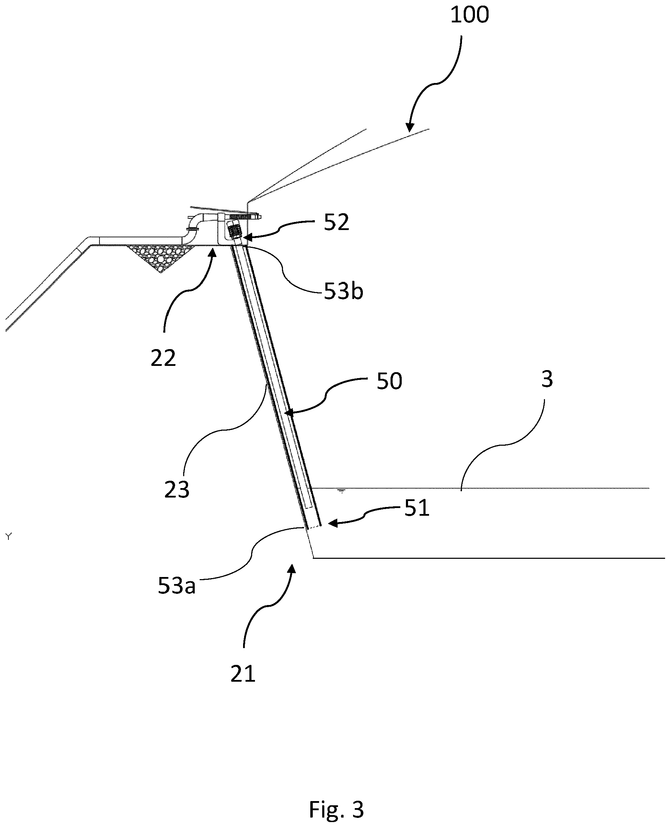

The extraction system comprises an extraction pipe 50 configured to transport the biomass 3 from inside the tank 30 to outside thereof. In other words, the extraction pipe 50 is configured to put the inside of the tank 30 in fluid communication with the outside. Preferably, the extraction pipe 50 comprises a hollow duct arranged inside the tank and immersed at least partially inside the biomass 3 arranged in the tank 30 and at least partially arranged outside the tank 30 and the biomass 3. More preferably, the extraction pipe 50 as well as the hollow duct has a biomass filling level substantially equal to a filling level of biomass 3 of the tank 30. In other words, in use, the insertion of the extraction pipe 50 inside the biomass 3 or the filling of the tank 30 with biomass 3 after positioning the extraction pipe 50 causes the gradual filling of the extraction pipe 50 with biomass. The biomass level inside the extraction pipe 50 thereby equals the biomass level inside the tank 30 (Figures 2 and 3).

The extraction pipe 50 extends between a first end portion 51 arranged proximate to the lower portion 21 and a second end portion 52 arranged proximate to the upper portion 22 along the inner surface 23 of the side wall 20 facing the inside of the tank 30. Preferably, the extraction pipe 50 is associated with the inner surface 23 of the side wall.

Advantageously, the arrangement of the extraction pipe 50 extended along the inner surface 23 eases the biomass extraction operations as well as the construction of the digester 1 and possible maintenance operations.

Advantageously, the arrangement of the extraction pipe 50 allows to easily integrate the system into the digester 1.

It is worth noting that this first end portion 51 is configured to be immersed within the biomass 3 contained in tank 30, while the second end portion 52 is outside the tank 30. Specifically, the first end portion 51 faces the bottom of the tank 31, preferably at the lower inner edge 27, while the second end portion 52 faces the outside of the tank 30 proximate to the supporting surface 29.

The extraction pipe 50 has an inlet opening 50a obtained proximate to the first end portion 51 and an outlet opening 50b obtained proximate to the second end portion 52. The inlet opening 50a is configured to allow the biomass to enter the extraction pipe 50, while the outlet opening 50b is configured to allow the biomass 3, sucked in by the pumping means along the extraction pipe 50, to exit.

Preferably, the inner surface 23 has a development profile between the lower portion 21 and the upper portion 22 of the side wall 20 defined by the inclination of the inner surface 23 with respect to the base 10. The development profile may, for example, be a plane inclined with respect to the base 10 or a plane perpendicular to the base itself. The extraction pipe 50 is configured to follow the development profile of the inner surface 23.

According to a preferred embodiment, the extraction pipe 50 is fixed to the inner surface 23 of the tank 30. According to a preferred aspect of the present invention, the extraction system 50 may comprise anchoring means configured to retain and reversibly secure, the extraction tube 50 to the inner surface 23.

According to a preferred embodiment, the extraction pipe 50 has a substantially straight development direction A-A along the inner surface 31 of the tank 30. Such straight development eases the extraction of the biomass and avoids unwanted accumulation of the biomass on any elbow of the extraction pipe 50.

According to a preferred embodiment, the extraction system 40 comprises a protection pipe 53 associated with the extraction pipe 50. Such protection pipe 53 is configured to surround the extraction pipe 50 along the inner surface 23. In other words, the extraction pipe 50 is inserted inside the protection pipe 53. The latter is associated with the inner surface 23 and extends between a first end portion 53a proximate to the lower portion 21 and a second end portion 53b proximate to the upper portion 22. Preferably, the protection pipe 53 is fixed to the inner surface 23 by anchoring means and the extraction pipe 40 is inserted inside the protection pipe 53 by the second end portion 53b. In other words, the protection pipe 53 defines a hollow insertion channel for the extraction pipe 50. Advantageously, the protection pipe 53 generates a "protected" environment from which the biomass can be sucked in. In addition, the protection pipe 53 allows the extraction pipe to be guided inside the tank 30.

It is worth noting that the material from which the protective pipe 53 is made can be PVC, PE, steel. Its extension along the inner surface 23 may be equal to a portion of the inner surface itself 23 or substantially to its full extension.

The extraction system comprises pumping means 60 associated with the extraction pipe 50 and configured to extract the biomass 3 from the tank 30 via the extraction pipe 50. Specifically, the pumping means 60 extract the biomass 3 by sucking it from the first end portion 51 and transporting it along the pipe 50 to the second end portion 52. In detail, the pumping means 60 in use are configured to extract the biomass contained inside the extraction pipe 50 and that which gradually replaces the biomass previously extracted within the pipe 50 during extraction by entering into the extraction pipe 50 from the first end portion 51. In other words, the pumping means 60 move the biomass inside the extraction pipe 50 for a path along the extraction pipe equal to the height difference between the biomass level inside the tank and substantially the second end portion 52 of the extraction pipe 50. Therefore, the work of the pumping means 60 is a function of the level of biomass 3 inside the tank as well as inside the extraction pipe 50 as the pumping media 60 have to overcome different pressure surges. For example, a high level of biomass i.e. proximate to the opening of the tank 30 (Figure 2) implies less work than a low level of biomass i.e. proximate to the tank base 31 (Figure 3).

Preferably, the pumping means 60 are associated with the second end portion 52 and are arranged externally to the tank 30 proximate to the upper portion 22. According to a preferred aspect of the present invention at least a portion of the pumping means 60 is external to the extraction pipe 50 and a portion of the pumping means 60 is internal to the extraction pipe proximate to the second end portion 52, preferably of the outlet opening 50b. Specifically, the pumping means 60 comprise a pump 61 whose motor is arranged outside the extraction pipe 50.

In detail, the extraction pipe 50 defines a suction pipe for pumping means 60 as well as for the pump 61.

According to preferred embodiment, the pumping means 60 comprise a vertical axis centrifugal pump 61 vertically aligned with the extraction pipe 50, preferably parallel to the development axis A-A of the extraction pipe 50. Preferably, the centrifugal pump has a variable flow rate depending on the volume of the digester and the volume of biomass to be handled.

In detail, the pumping means 60, in use, suck in the biomass 3 contained in the extraction pipe 50, which gradually re-enters it from the first inlet opening 50a immersed in the biomass. If the protective pipe 53 is present, the pumping means 60 consequently also suck in the biomass contained in the protective pipe.

Thanks to the positioning of the pumping means 60 outside the tank it is possible for operators to easily access the pump 61 for maintenance and replacement. It is also worth noting that the extraction pipe 50 may be extracted as required from the tank 30 at the upper portion 22 from the second end portion 52 to ease maintenance or replacement operations also in this case.

The digester 1 comprises a platform 70 attached to the side wall 20 at the upper portion 22 and at least partially suspended cantilevered towards the inside of the tank 30. The platform 70 projects from the upper inner edge 25 towards the inside of the tank 30. Such platform 70 is configured to retain the extraction pipe 50 on the cantilevered portion proximate to the second end portion 52. Preferably, the second end portion 52 projects from the platform 70 to the outside of the tank 30. More preferably, the platform 70 has a passage opening within which the extraction pipe 50 is inserted and fixed proximate to the second end portion 52. Specifically, the hollow duct is inserted into the passage opening and is retained outside by the platform itself. The pipe may thereby be pulled out and inserted as required. Thanks to the platform 70, it is therefore possible to ease operations on the extraction pipe 50 such as maintenance, control and routine operations. Preferably, the platform 70 eases the correct positioning of the extraction pipe 50 along the inner surface of the side wall 20.

It should be noted that the platform 70 also allows to support the pumping means 60 to ease the relative access. The pumping means 60 are thereby accessible from the platform 70. Preferably, the platform 70 retaining the extraction pipe 50 at the second end portion may ease the assembly of the pumping means 60 in the relative seat of the extraction pipe 50.

It is worth noting that, if present, the protective pipe 53 is associated with the cantilevered portion of the platform 70 inside the tank 30. Specifically, the extraction pipe 53 extends from the platform 70 towards the tank base 31 along the inner surface 23 and is attached to the platform 70 at the second end portion 53b. Thereby, the extraction pipe 50 passing through the passage opening enters directly into the protection pipe 53.

Advantageously, the cantilevered extension is particularly advantageous for installing the extraction pipe 50 as well as for using active components on the platform where the at least one active component is, for example, one or more of an irrigation apparatus 80, a monitoring and sensor system, a substrate recovery system, a gas recovery system or a stirring system, a mixer, a heating system, a desulphurisation system.

According to a preferred embodiment, the platform 70 comprises a fastening portion 71 for fastening the platform 70 itself to the side wall 20 of the digester 1, preferably to the supporting surface 29 so that it is at least partially cantilevered towards the inside of the tank 30. Specifically, the platform 70 comprises fastening means for fastening the fastening portion 71 to the side wall 20. Such fastening means are adapted to keep the fastening portion 71 adhered to the supporting surface 29 of the side wall 20. For example, the fastening means include a counterweight and/or anchoring elements configured to be active within the side wall 20 to support the platform itself.

Preferably, the platform 1 comprises an operating portion 72 connected to the fastening portion 71 and arranged cantilevered within the tank. This eases the action on the biomass 3 by means of the extraction pipe 50 as well as its movement within the digester tank using the irrigation apparatus 80.

Such operating portion 72 comprises sealing means adapted to retain the second end portion 52 and coupling means configured to house and support at least one active component of the digester. In addition, the operating portion 72 comprises anchoring means for a digester tank cover membrane 100, described hereinafter. It is worth noting that the operating portion 73 also comprises sealing means adapted to retain the second end portion 53b of the protective pipe 53, if present.

According to a preferred aspect of the present invention, the operating portion 72 comprises a first plate 73 adapted to be parallel to the base of the tank 30 and a second plate 74 transverse to the first plate 73. This first plate 73 connects the fastening portion 71 and the second plate 74 of the operating portion 72. Preferably, the second plate 73 of the operating portion 72 is orthogonal to the first plate 31. Still preferably, the digester membrane 100 is connected to the platform 70 at the second plate 74 of the operating portion 3. Specifically, the first plate 73 extends from the inner upper edge 25 so as to protrude into the tank parallel to the tank base 31. This first plate 72 comprises the sealing means for both the extraction pipe 50 and the protection pipe 53, while the second plate 74 comprises the coupling means.

It is worth noting that the platform 70 also allows to associate safety devices proximate thereto in order to make operations on the digester safe for the operator.

According to a preferred embodiment, the digester 1 comprises an irrigation apparatus 80 configured to spray biomass via pumping means 60 within the tank 30. Preferably, the irrigation apparatus 80 may spray biomass at least partly extracted from the extraction system 40 and/or fresh biomass that may receive as explained hereinafter from a biomass input apparatus 90.

More preferably, the pumping means 60 are associated with the irrigation apparatus 80 and are configured to extract the biomass 3 and reintroduce it via the irrigation apparatus 80. Specifically, the biomass extraction system 40 is associated with the irrigation apparatus 80 to move the biomass 3 present inside the tank 30. Alternatively or in combination, the irrigation apparatus 80 may input fresh biomass or combine fresh biomass with the extracted one.

Preferably, the irrigation apparatus 80 is associated with the platform 70. More preferably, the irrigation apparatus 80 is associated with the platform 70 at the active portion 72 by means of the coupling means. Advantageously, the irrigation apparatus 80 is arranged on the cantilevered portion of the platform and allows to act on the biomass from above the tank opening along a vertical direction orthogonal to the bottom of the tank 30. In other words, the irrigation apparatus 80 sprays the biomass it receives into the tank 30 on the previous biomass present in the tank 30 or on the tank base 31 in case the tank was previously empty.

More preferably, the irrigation apparatus 80 comprises a nozzle 81 facing the inside of the tank 30 and configured to spray biomass. Such nozzle 81 extends from the active portion 72 preferably from the second plate 74 towards the inside of the tank 50. Specifically, the nozzle 81 extends from an inner surface of the second plate 74 towards the inside of the tank preferably cantilevered from the surface and is fixed to the second plate 74 by coupling means such as bolts or welds.

The irrigation apparatus 80 further comprises a connecting channel 82 configured to place in fluid communication the nozzle 81 and the second end portion 52 of the extraction pipe 50, preferably the outlet opening 50b. Specifically, the connecting channel 82 extends between the second end portion 52, preferably outside the tank 30, and the second plate 74 at the coupling between the nozzle 81 and the second plate. In detail, the nozzle 81 extends between a first end coupled to the second plate 74 and configured to receive biomass and an opposite second free end configured to deliver biomass. The connecting channel 82 places the second end 52, as well as the hollow channel, in fluid communication with the first end of the nozzle 80.

The biomass extracted by the extraction system 40 may thereby be diverted to the connecting channel 82 and re-introduced into the tank via the nozzle 81 generating a recirculation of the biomass between that facing the tank base 31 and that facing the tank opening 30.

According to a preferred embodiment, the digester 1 comprises a biomass input apparatus 90 configured to add additional biomass within the tank 30 and/or to the biomass extracted by means of the extraction system 40 via the irrigation apparatus 80. Specifically, the biomass input apparatus 90 is configured to add fresh biomass inside the tank 30 either directly or by mixing proximate to the nozzle 81 with the biomass extracted before entering the tank.

Preferably, the input apparatus 90 comprises a reservoir 91 of fresh biomass external to the tank 30, a connecting circuit 92 configured to place the reservoir 91 in fluid communication with the connecting channel 82 upstream of the nozzle 81. The fresh biomass may thereby be brought up to the nozzle 81 and input into the tank. More preferably, the input apparatus 90 comprises additional pumping means associated with the connection circuit 92 and configured to transport fresh biomass from the reservoir 91 to the irrigation apparatus 80. It is worth noting that the connecting circuit 92 is associated with the outer surface 24 of the side wall 20. Specifically, the connecting circuit 92 runs along the outer surface 24 from the reservoir 91 to the irrigation apparatus 80.

According to a preferred embodiment, the tank 30 is provided with a cover membrane 100 mechanically fixed to the side wall 30 and preferably to the platform 70 at the active portion 72. Specifically, the cover membrane 100 defines with its associated tank 30 a digester reservoir . Alternatively or in combination with the aforesaid embodiment, the cover membrane 100 defines with the tank 30 a storage reservoir. Such cover membrane 100 is configured to collect the biogas generated by the organic matter contained within the tank. Specifically, the biogas flowing out of the organic matter is enclosed between the latter and the cover membrane 100.

It is worth noting that the digester 1 may comprise one or more extraction systems 50 and their associated platforms 70, irrigation apparatus 80 and input apparatus 90 as described above.

It is a further object of the present invention a biogas production plant provided with one or more digesters 1. The plant comprises at least one digester reservoir and one or more circuits configured to move the biogas produced from the digester reservoir to the users.

## Claims

1. Digester for biogas production (1), comprising:
- a base (10) associated with a supporting ground (2)
- a side wall (20) extended between a lower portion (21) associated with the base (10) and an upper portion (22) spaced from the lower portion (21) along a height direction (X-X) perpendicular to the base (10), said side wall (20) with the base (10) defining a tank (30) configured to receive inside it a quantity of biomass (3) for generating biogas;
- a biomass extraction system (40) configured to transport the biomass (3) from the tank (30), the extraction system (40) comprising
- an extraction pipe (50) configured to transport the biomass (3) from inside the tank (30) to outside the tank, said extraction pipe (50) extending between a first end position (51) arranged near the lower portion (21) and a second end position (52) in proximity to the upper portion (22);
- pumping means (60) associated with the extraction pipe (50) and configured to extract the biomass (3) from the tank (30) via the extraction pipe (50);
**characterised in that** the digester (1) comprises a platform (70) fixed to the side wall (20) at the upper portion (22) and at least partially suspended cantilevered towards the inside of the tank (30), said platform (70) being configured to retain the extraction pipe (50) in proximity to the second end portion (52),
**and wherein**
the extraction pipe (50) extends between the first end portion (51) and the second end portion (52) retained by the platform (70) along an inner surface (23) of the side wall (20) facing the inside of the tank (30).

2. Digester (1) in accordance with claim 1, wherein the inner surface (23) of the side wall (20) has a development profile between the lower portion (21) and the upper portion (22) of the side wall (20), the extraction pipe (50) being configured to follow the development profile.

3. Digester (1) in accordance with claim 1 or 2, wherein the extraction pipe (50) has a substantially straight development direction (A-A) along the inner surface (31) of the side wall (20).

4. Digester (1) in accordance with any one of claims 1 to 3, wherein the extraction system (40) comprises a protection pipe (53) associated with the extraction pipe (50) and configured to surround it along the inner surface (23).

5. Digester (1) in accordance with any one of claims 1 to 4, wherein the pumping means (60) are associated with the second end portion (52) and are arranged externally to the tank (30) in proximity to the upper portion (22).

6. Digester (1) in accordance with any one of claims 1 to 5, wherein the pumping means (60) comprise a vertical axis centrifugal pump aligned with the extraction pipe (50).

7. Digester (1) in accordance with any one of claims 1 to 6, comprising an irrigation apparatus (80) configured via the pumping means (60), to spray fresh biomass or a combination thereof which was at least partially extracted by the extraction system (40) into the tank (30).

8. Digester (1) in accordance with claim 7, wherein the irrigation apparatus (80) comprises:
- a nozzle (81) facing the inside of the tank (30) and configured to spray biomass;
- a connecting channel (82) configured to place the nozzle (81) and the second end portion (52) of the extraction pipe (50) in fluid communication.

9. Digester (1) in accordance with claim 7 or 8, comprising a biomass input apparatus (90) configured to add additional biomass inside the tank (30) and/or to the biomass extracted by means of the extraction system (40) via the irrigation apparatus (80).

## Patentansprüche

1. Faulbehälter für Biogaserzeugung (1), umfassend:
- eine Basis (10), die mit einem tragenden Boden (2) assoziiert ist
- eine Seitenwand (20), die sich zwischen einem unteren Abschnitt (21), der mit der Basis (10) assoziiert ist, und einem oberen Abschnitt (22) erstreckt, der von dem unteren Abschnitt (21) entlang einer Höhenrichtung (X-X) senkrecht zu der Basis (10) beabstandet ist, wobei die Seitenwand (20) mit der Basis (10) einen Tank (30) definiert, der konfiguriert ist, um darin eine Menge an Biomasse (3) zum Erzeugen von Biogas aufzunehmen;
- ein Biomasseextraktionssystem (40), das konfiguriert ist, um die Biomasse (3) aus dem Tank (30) zu transportieren, das Extraktionssystem (40) umfassend:
- ein Entnahmerohr (50), das konfiguriert ist, um die Biomasse (3) aus dem Inneren des Tanks (30) nach außerhalb des Tanks zu transportieren, wobei sich das Entnahmerohr (50) zwischen einer ersten Endposition (51), die in der Nähe des unteren Abschnitts (21) angeordnet ist, und einer zweiten Endposition (52) in der Nähe des oberen Abschnitts (22) erstreckt;
- Pumpeinrichtungen (60), die mit dem Entnahmerohr (50) assoziiert und konfiguriert sind, um die Biomasse (3) über das Entnahmerohr (50) aus dem Tank (30) zu entnehmen;
**dadurch gekennzeichnet, dass** der Faulbehälter (1) eine Plattform (70) umfasst, die an dem oberen Abschnitt (22) an der Seitenwand (20) befestigt und zumindest teilweise freitragend zum Inneren des Tanks (30) aufgehängt ist, wobei die Plattform (70) konfiguriert ist, um das Entnahmerohr (50) in der Nähe des zweiten Endabschnitts (52) zu halten,
**und wobei**
sich das Entnahmerohr (50) zwischen dem ersten Endabschnitt (51) und dem zweiten Endabschnitt (52), der von der Plattform (70) gehalten wird, entlang einer Innenfläche (23) der Seitenwand (20), die dem Inneren des Tanks (30) zugewandt ist, erstreckt.

2. Faulbehälter (1) nach Anspruch 1, wobei die Innenfläche (23) der Seitenwand (20) ein Verlaufsprofil zwischen dem unteren Abschnitt (21) und dem oberen Abschnitt (22) der Seitenwand (20) aufweist, wobei das Entnahmerohr (50) konfiguriert ist, um dem Verlaufsprofil zu folgen.

3. Faulbehälter (1) nach Anspruch 1 oder 2, wobei das Entnahmerohr (50) eine im Wesentlichen gerade Verlaufsrichtung (A-A) entlang der Innenfläche (31) der Seitenwand (20) aufweist.

4. Faulbehälter (1) nach einem der Ansprüche 1 bis 3, wobei das Entnahmesystem (40) ein Schutzrohr (53) umfasst, das mit dem Absaugrohr (50) assoziiert und konfiguriert ist, um es entlang der Innenfläche (23) zu umgeben.

5. Faulbehälter (1) nach einem der Ansprüche 1 bis 4, wobei die Pumpeinrichtungen (60) mit dem zweiten Endabschnitt (52) assoziiert sind und in der Nähe des oberen Abschnitts (22) außerhalb des Tanks (30) angeordnet sind.

6. Faulbehälter (1) nach einem der Ansprüche 1 bis 5, wobei die Pumpeinrichtungen (60) eine Vertikalachsen-Zentrifugalpumpe umfassen, die mit dem Entnahmerohr (50) ausgerichtet ist.

7. Faulbehälter (1) nach einem der Ansprüche 1 bis 6, umfassend eine Bewässerungsvorrichtung (80), die über die Pumpeinrichtungen (60) konfiguriert ist, um frische Biomasse oder eine Kombination davon, die zumindest teilweise durch das Entnahmesystem (40) entnommen wurde, in den Tank (30) zu sprühen.

8. Faulbehälter (1) nach Anspruch 7, wobei die Bewässerungsvorrichtung (80) Folgendes umfasst:
- eine Düse (81), die dem Inneren des Tanks (30) zugewandt und konfiguriert ist, um Biomasse zu sprühen;
- einen Verbindungskanal (82), der konfiguriert ist, um die Düse (81) und den zweiten Endabschnitt (52) des Entnahmerohrs (50) in Fluidverbindung zu bringen.

9. Faulbehälter (1) nach Anspruch 7 oder 8, umfassend eine Biomasseeingabevorrichtung (90), die konfiguriert ist, um zusätzliche Biomasse innerhalb des Tanks (30) und/oder zu der mittels des Entnahmesystems (40) über die Bewässerungsvorrichtung (80) entnommenen Biomasse hinzuzufügen.

## Revendications

1. Digesteur destiné à la production de biogaz (1), comprenant :
- une base (10) associée à un sol de support (2)
- une paroi latérale (20) s'étendant entre une partie inférieure (21) associée à la base (10) et une partie supérieure (22) espacée de la partie inférieure (21) le long d'un sens de la hauteur (X-X) perpendiculaire à la base (10), ladite paroi latérale (20) avec la base (10) définissant un réservoir (30) configuré pour recevoir en son sein une quantité de biomasse (3) pour générer du biogaz ;
- un système d'extraction de biomasse (40) configuré pour transporter la biomasse (3) depuis le réservoir (30), le système d'extraction (40) comprenant
- un tuyau d'extraction (50) configuré pour transporter la biomasse (3) de l'intérieur du réservoir (30) à l'extérieur du réservoir, ledit tuyau d'extraction (50) s'étendant entre une première position d'extrémité (51) agencée à proximité de la partie inférieure (21) et une seconde position d'extrémité (52) à proximité de la partie supérieure (22) ;
- des moyens de pompage (60) associés au tuyau d'extraction (50) et configurés pour extraire la biomasse (3) du réservoir (30) via le tuyau d'extraction (50) ;
**caractérisé en ce que** le digesteur (1) comprend une plate-forme (70) fixée à la paroi latérale (20) au niveau de la partie supérieure (22) et au moins partiellement suspendue en porte-à-faux vers l'intérieur du réservoir (30), ladite plate-forme (70) étant configurée pour retenir le tuyau d'extraction (50) à proximité de la seconde partie d'extrémité (52),
**et dans lequel**
le tuyau d'extraction (50) s'étend entre la première partie d'extrémité (51) et la seconde partie d'extrémité (52), retenu par la plate-forme (70) le long d'une surface interne (23) de la paroi latérale (20) tournée vers l'intérieur du réservoir (30).

2. Digesteur (1) selon la revendication 1, dans lequel la surface interne (23) de la paroi latérale (20) présente un profil de développement entre la partie inférieure (21) et la partie supérieure (22) de la paroi latérale (20), le tuyau d'extraction (50) étant configuré pour suivre le profil de développement.

3. Digesteur (1) selon la revendication 1 ou 2, dans lequel le tuyau d'extraction (50) présente une direction de développement sensiblement droite (A-A) le long de la surface interne (31) de la paroi latérale (20).

4. Digesteur (1) selon l'une quelconque des revendications 1 à 3, dans lequel le système d'extraction (40) comprend un tuyau de protection (53) associé au tuyau d'extraction (50) et configuré pour l'entourer le long de la surface interne (23).

5. Digesteur (1) selon l'une quelconque des revendications 1 à 4, dans lequel les moyens de pompage (60) sont associés à la seconde partie d'extrémité (52) et sont agencés à l'extérieur du réservoir (30) à proximité de la partie supérieure (22).

6. Digesteur (1) selon l'une quelconque des revendications 1 à 5, dans lequel les moyens de pompage (60) comprennent une pompe centrifuge à axe vertical alignée sur le tuyau d'extraction (50).

7. Digesteur (1) selon l'une quelconque des revendications 1 à 6, comprenant un appareil d'irrigation (80) configuré via les moyens de pompage (60), pour pulvériser de la biomasse fraîche ou une combinaison de celle-ci qui a été au moins partiellement extraite par le système d'extraction (40) dans le réservoir (30).

8. Digesteur (1) selon la revendication 7, dans lequel l'appareil d'irrigation (80) comprend :
- une buse (81) tournée vers l'intérieur du réservoir (30) et configurée pour pulvériser de la biomasse ;
- un canal de raccordement (82) configuré pour mettre la buse (81) et la seconde partie d'extrémité (52) du tuyau d'extraction (50) en communication fluidique.

9. Digesteur (1) selon la revendication 7 ou 8, comprenant un appareil d'entrée de biomasse (90) configuré pour rajouter de la biomasse à l'intérieur du réservoir (30) et/ou à la biomasse extraite au moyen du système d'extraction (40) via l'appareil d'irrigation (80).
